# EUROPEAN PATENT SPECIFICATION

(11) **EP 1 861 149 B1**
(45) Date of publication and mention of the grant of the patent: **15.07.2015**
(21) Application number: 06723725.5
(22) Date of filing: 21.03.2006
(51) Int. Cl.: A61M 15/00

(54) **Apparatus for the administration of pharmaceutical products in aerosol form**
Gerät zur Verabreichung von pharmazeutischen Produkten in Aerosolform
Appareil pour l'administration de produits pharmaceutiques sous forme d'aérosol

(30) Priority: 24.03.2005 IT MI20050491
(43) Date of publication of application: 05.12.2007
(73) Proprietor: IPH Establishment, 6460 Vaduz (LI)
(72) Inventor: LICCIULLI, Paolo, I-20025 Legnano (IT)
(74) Representative: Martegani, Franco
(86) International application number: PCT/EP2006/002746
(87) International publication number: WO 2006/100102

(56) References cited:
- WO-A-03/084591
- US-A- 2 566 806
- US-A1- 2002 053 345
- US-A1- 2003 168 058

## Description

The present invention relates to an apparatus for the administration of pharmaceutical products in aerosol form or nebulized.

As is known, the respiratory system, comprising lungs and chest, operates as a pump which rests on a muscle called diaphragm, which coordinates the inspiration and expiration actions of air and oxygen. In the natural respiratory process, the contraction of the diaphragm generates a vacuum in the lungs, which causes the inspiration of air from the outside.

As is known, there are numerous affections of the respiratory system which diminish the respiratory capacity of the patient.

In the case, for example, of patients suffering from asthma or one of the many other lung affections, which require the administration of suitable curative products in the bronchi and lung alveoli, it should be remembered that, in effecting said administration, there is a clear decrease in the respiratory capacity of the patient.

One known solution for curing sinusitis by delivering a drug to a patient is known from document US2,566,806, that shows an apparatus provided by two rubber bulb which can be squeezed by an user in order to provide two distinct actions, an action of subministration of the drug and consequent suction action.

This device, even if useful for treatment of sinusitis, suffers some drawbacks when used administering drugs to the bronchi of a patient.

First of all, the two rubber bulbs do not allow for a continuous action and cannot be used simultaneously.

Besides this, the suction action does not allow to perform a useful drug administration in patients suffering a decrease in the respiratory capacity.

There are currently three main methods for the administration of aerosol or drug treatment to these patients, i.e. (1) by means of atomizers of the Venturi-jet type or of the ultrasonic piezoelectric type, which produce aerosols from drug solutions; (2) by means of inhalators of measured dosages (MDI) consisting of pressurized cylinders with fluorocarbides or other gases and (3) dry powder inhalators.

Devices of type (1) or moist atomizers are jet nebulizers which exploit the Venturi principle: the energy source is compressed air which also serves to direct the aerosol towards the spontaneous respiration of the patient. In jet nebulizers, the oxygen and/or air flow creates the aerosol, through the device, starting from the drug in solution and carries it towards the patient, who can then breathe it from a mask or mouthpiece.

The use of the respiratory tract for the administration of drugs is becoming increasingly more important. In this type of approach, not only has the application of drug products which produce local effects in the treatment of lung affections been developed, but new strategies have also been envisaged, which use the lungs as the organ through which the drugs enter the body and produce systemic effects.

The use of this system, inhalation, for the administration of drugs has led to an ever-growing demand for an improvement in the quality of inhalation, which is not sufficient with the devices currently existing on the market.

At present, in fact, devices of the jet nebulizer type are absolutely inefficient as only 20% of the product, in the form of an aerosol, reaches the inner recesses of the lungs.

This is due to the fact that the efficiency of the existing devices depends on the conditions of the patient and on his respiratory capacity. It is the patient, in fact, that carries the product, nebulized by a pump starting from a liquid in a Venturi basin, towards the aerial tract, by means of his respiratory capacity. It is evident that a patient with a reduced respiratory capacity cannot effect a sufficient transmission of the nebulized drug.

As mentioned above, the penetration of the nebulized product is limited and 80% of the drug remains in the first respiratory tract.

It is evident that, if the pathology to be cured is a pathology of the first respiratory tract, the efficiency is good, otherwise the aerosol treatment is practically null.

Furthermore, it should be remembered that the rest of the vapour passes from the throat to the stomach, with relevant side-effects in the case of the use of particular active principles.

An increase in the dosage in order to compensate the inefficiency of the nebulizer can be harmful for the patient's health.

A general objective of the present invention is therefore to solve the above-mentioned drawbacks.

An object of the present invention is an apparatus for the administration of pharmaceutical products in the form of an aerosol, comprising means for generating a constant air flow; means for generating a pre-determined air flow; means for conveying the air flow to the aerial tracts of a patient; means for containing the pharmaceutical product to be nebulized, said equipment being characterized in that it includes means for generating a pre-established air flow during all expiration phases of the respiratory cycle, said means for generating a pre-established air flow being in pneumatic connection with the means for conveying the air flow.

The means for generating a constant air flow consist of a pump and the means for generating a pre-established air flow during all expiration phases also consist of a resistance pump which synchronizes the administration of a pre-established flow of air with the expiration phase.

The apparatus according to the present invention can also be used without a pharmaceutical product.

The means for conveying the air flow to the aerial tracts of a patient comprise a connector and a mask or mouthpiece, said means for generating a pre-established flow of air being operatively connected to said connector or to said mask or mouthpiece, by the means for conveying the air flow.

In particular, the first pump has the objective of nebulizing the drug as a common aerosol, the second, on the contrary, has the objective of supplying a flow of air which, at the right moment, intervenes on the normal ventilation flow by varying its rate and resistances with the purpose of obtaining a greater and better penetration of the drug towards the small aerial tract and stimulating a movement of the large aerial tracts so as to favour expectoration.

The main advantage of the apparatus according to the invention, in fact, consists not only in allowing therapies similar to those of the aerosol apparatuses already on the market to be effected, i.e. inhalation therapies effective for the first aerial tract, but also providing inhalation therapies effective towards the small aerial tracts.

Thanks to the improved efficiency of the apparatus, only partially dependent on the patient's respiratory capacity, it is also evident that lower dosages of the drug can be used, which will be better distributed.

A further advantage of the apparatus according to the present invention, moreover, is that it also effects a lung exercise, which is already effective even without drugs.

By exploiting the elastic capacities of the aerial tracts, by the use of a resistant pressure in the expiration phase, the apparatus according to the present invention compels the aerial tracts to slightly dilate until the end of the expiration. At the end of this phase, the resistant pressure is interrupted, thus relaxing the walls of the respiratory tracts: (the resistant pressure is interrupted at any point of the expiration phase, according to necessity).

This movement facilitates the inlet of air in the subsequent inspiration phase. The sequence of these movements allows a better change of air, the detachment of mucus and secretions, as well as the penetration of the drug.

It is also a "natural" movement, which follows the physiological characteristics of the patient's respiration, as the functioning of the apparatus is regulated by the manner and frequency of the patient's respiration.

The structural and functional characteristics of the present invention and its advantages with respect to the known art will appear even more evident by examining the following description referring to the enclosed drawings, in which:
figure 1 is a schematic representation of an embodiment of the apparatus according the present invention;
figure 2 is a schematic representation of the pressure variation with the variation in time in an apparatus such as that represented in figure 1;
figure 3 is a schematic representation of the pressure variation in relation to time in a respiration cycle of a patient subjected to treatment with the apparatus according to the present invention, which envisages the first resistant pump only;
figure 4 is a schematic representation of the pressure variation in relation to time in a respiration cycle of a patient subjected to treatment with the apparatus according to the embodiment of the present invention, which envisages also the resistant pump operating in continuous.

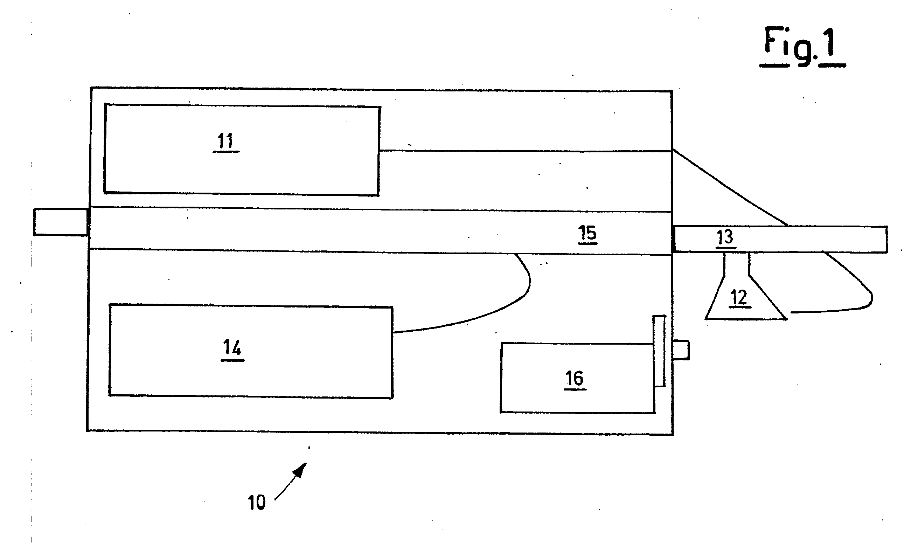

In particular, figure 1 shows an embodiment of the apparatus according to the present invention, wherein 10 indicates the apparatus. It includes a nebulization pump 11 which generates a constant air flow, which effects the nebulization of the liquid solution of the pharmaceutical product contained in 12, i.e. in a container consisting of a Venturi basin. More specifically, the constant air flow generated by the pump 11, is directly sent to the nebulization basin 12.

This basin is then connected, through a tubular connection 13, to a mask or mouthpiece (not shown in the figure), worn by the patient and through which the nebulized product enters the aerial tract of the patient.

The apparatus 10, in the embodiment shown in figure 1, also envisages a second pump indicated as 14, connected, through suitable means 15 for conveying the air flow, to the aerial tracts of a patient. This second pump 14 generates a resistant pressure, supplied by means of a flow of compressed air, contrary in the expiration phase. The means 15 for conveying the flow of air to the aerial tracts of the patient is connected, again through the tubular connection 13, to the mask or mouthpiece (not shown in the figure) worn by the patient and through which the nebulized product enters the aerial tracts of the patient.

The apparatus shown in figure 1 also includes a handling and control system of the pumps 16, which, in the embodiment represented in the figure, is a potentiometer.

The air flow generated by the pumps used in the apparatus according to the present invention is preferably a flow of 10 liters per minute.

In particular, the means for conveying the air flow to the patient 15 is pneumatically connected with the fitting 13 and then with the mask, so as to administrate a certain quantity of air in the expiration flow. The air expired by the patient passes through the fitting 13 to the duct 15.

The patient consequently breathes naturally in all phases of the respiratory cycle, receiving a pressure of positive air supplied by the apparatus due to the action of the pump 11 with the nebulized pharmaceutical product, during the inspiration phase and a pressure of positive air during the expiration phase due to the combined action of the pump 11 and the resistant pump 14.

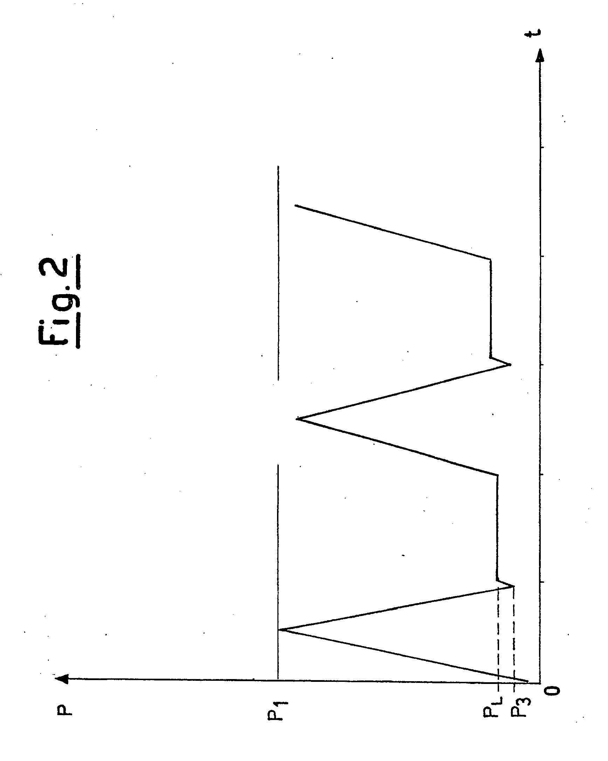

The apparatus according to the present invention, schematically shown in figure 1, shows a pressure variation in relation to the time in a respiratory cycle with alternating inspiration and expiration phases, said variation being schematically shown in figure 2.

In particular, in the apparatus 1, P₁ represents the pressure at the beginning of the expiration phase, whereas the final pressure of the expiration phase P₃ is lower than the pressure P₂ of the beginning of the subsequent inspiration phase.

The slight depression caused by the pressure drop between P₂ and P₃ is sufficient for creating a vortex in the aerial tracts of the patient, which allows the air flow of the subsequent inspiration phase to also reach the most peripheral aerial tracts, i.e. the small aerial tracts. This pressure drop is generated by the switching off of the resistant pump 14.

In this way the apparatus 1 according to the present invention, by exploiting the elastic properties of the aerial tracts, with the use of a resistant pressure during the expiration phase, forces a slight dilatation upon the aerial tracts, which reaches the end of the expiration. At the end of this phase, the resistant pressure is interrupted, thus relaxing the respiratory tracts.

This movement facilitates the inlet of air during the subsequent inspiration phase. The succession of these movements allows a better change of air, the detachment of mucus and secretions and penetration of the drug. A better penetration of the pharmaceutical product towards the small aerial tracts is in fact obtained together with a stimulation of movement in the large aerial tracts, thus favouring expectoration.

Thanks to this movement, the apparatus according to the present invention also effects a lung exercise which is already efficacious even without drugs.

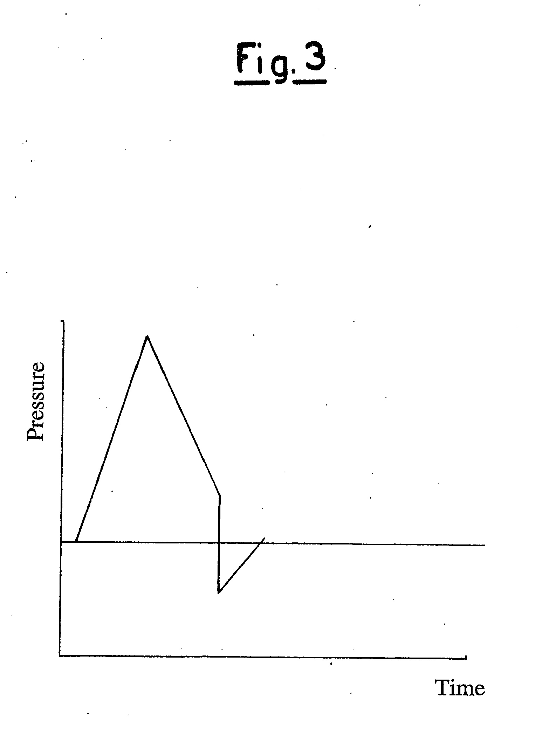

Fig. 3 shows the pressure variation in relation to the time in a respiration cycle of a patient subjected to treatment with the apparatus according to the present invention, which includes a single resistant pump. In particular, it shows the pressure variation per volume of air (from 0 to 10 cm of water), wherein the respiration pressure and the counter-pressure drop are evident.

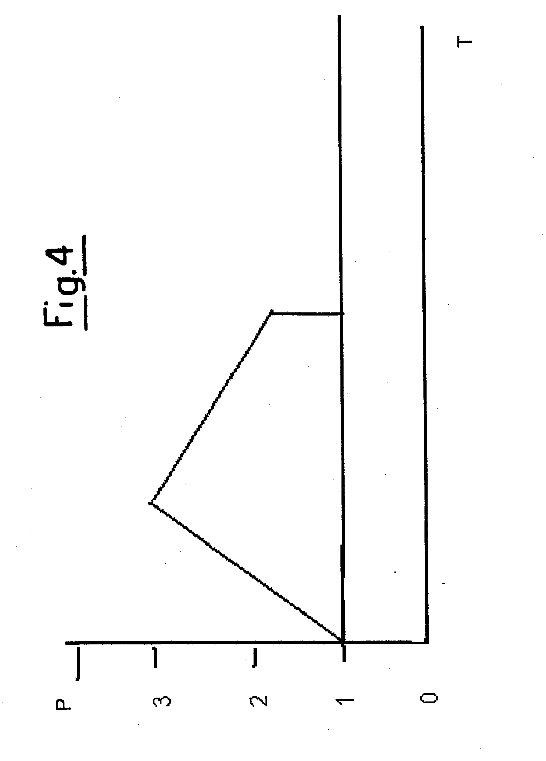

Fig. 4 shows the pressure variation in relation to the time in a respiration cycle of a patient subjected to treatment with the apparatus according to the embodiment of the present invention which includes a second resistant pump. In particular, the pressure variation is shown per volume of air (from 1 to 10 cm of water), wherein the respiration pressure and the counter-pressure drop are evident.

## Claims

1. An apparatus for the administration of pharmaceutical products in the form of an aerosol, comprising:
- means for generating a constant flow of air;
- means for generating a pre-established flow of air;
- means for conveying the air flow to the aerial tracts of a patient,
- means for containing the pharmaceutical product to be nebulized (12),
said means for containing the pharmaceutical product to be nebulized (12) being connected to a mask or mouth-piece,
said means for generating a constant flow of air are a nebulisation pump (11) connected to said means for containing the pharmaceutical product to be nebulised (12), said apparatus being **characterized in that** said means for generating a pre-established flow of air is a resistant pump (14) connected to said means for conveying the air flow to the aerial tracts of a patient (15),
said nebulisation pump (11) being active during both an inspiration phase and an expiration phase of the patient to supply a pressure of positive air,
said resistant pump (14) being active during said expiration phase of the patient for generating a flow of contrary compressed air.

2. The apparatus according to claim 1, **characterized in that** said resistant pump (14) synchronizes the administration of a pre-established air flow with the expiration phase.

3. The apparatus according to claim 1, **characterized in that** the means for conveying the air flow to the aerial tracts of a patient (15) comprise a connector and a mask or mouthpiece, said means for conveying a pre-established air flow being operatively connected to said connector or to said mask or mouthpiece through the means for conveying the air flow.

## Patentansprüche

1. Vorrichtung zum Verabreichen pharmazeutischer Produkte in der Form eines Aerosols, umfassend:
- ein Mittel zum Erzeugen einer konstanten Luftströmung;
- ein Mittel zum Erzeugen einer vorher festgesetzten Luftströmung;
- ein Mittel zum Fördern der Luftströmung zu den Luftwegen eines Patienten,
- ein Mittel zum Enthalten des pharmazeutischen Produktes, das zu vernebeln ist, (12),
wobei das Mittel zum Enthalten des pharmazeutischen Produktes, das zu vernebeln ist, (12) mit einer Maske oder einem Mundstück verbunden ist, das Mittel zum Erzeugen einer konstanten Luftströmung eine Vernebelungspumpe (11) ist, die mit dem Mittel zum Enthalten des pharmazeutischen Produktes, das zu vernebeln ist, (12) verbunden ist, wobei die Vorrichtung **dadurch gekennzeichnet ist, dass**
das Mittel zum Erzeugen einer vorher festgesetzten Luftströmung eine widerstandsfähige Pumpe (14) ist, die mit dem Mittel zum Fördern der Luftströmung zu den Luftwegen eines Patienten (15) verbunden ist,
wobei die Vernebelungspumpe (11) während sowohl einer Einatmungsphase als auch einer Ausatmungsphase des Patienten aktiv ist, um einen Druck von positiver Luft zu liefern,
wobei die widerstandsfähige Pumpe (14) während der Ausatmungsphase des Patienten zur Erzeugung einer Strömung von entgegengesetzt gerichteter Druckluft aktiv ist.

2. Vorrichtung nach Anspruch 1, **dadurch gekennzeichnet, dass** die widerstandsfähige Pumpe (14) die Verabreichung einer vorher festgesetzten Luftströmung mit der Ausatmungsphase synchronisiert.

3. Vorrichtung nach Anspruch 1, **dadurch gekennzeichnet, dass** das Mittel zum Fördern der Luftströmung zu den Luftwegen eines Patienten (15) einen Verbinder und eine Maske oder ein Mundstück umfasst, wobei das Mittel zum Fördern einer vorher festgesetzten Luftströmung funktional mit dem Verbinder oder der Maske oder dem Mundstück durch das Mittel zum Fördern der Luftströmung verbunden ist.

## Revendications

1. Appareil pour l'administration de produits pharmaceutiques sous forme d'aérosol, comprenant :
- des moyens pour produire un flux constant d'air ;
- des moyens pour produire un flux préétabli d'air ;
- des moyens pour transporter le flux d'air dans les voies aériennes d'un patient,
- des moyens pour contenir le produit pharmaceutique à nébuliser (12), lesdits moyens pour contenir le produit pharmaceutique à nébuliser (12) étant reliés à un masque ou à un embout buccal,
lesdits moyens pour produire un flux constant d'air sont une pompe de nébulisation (11) reliée auxdits moyens pour contenir le produit pharmaceutique à nébuliser (12),
ledit appareil étant **caractérisé en ce que**
lesdits moyens pour produire un flux préétabli d'air sont une pompe de résistance (14) reliée auxdits moyens pour transporter le flux d'air dans les voies aériennes d'un patient (15),
ladite pompe de nébulisation (11) étant active à la fois durant une phase d'inspiration et une phase d'expiration du patient pour fournir une pression d'air positif,
ladite pompe de résistance (14) étant active durant ladite phase d'expiration du patient pour produire un flux d'air comprimé contraire.

2. Appareil selon la revendication 1, **caractérisé en ce que** ladite pompe de résistance (14) synchronise l'administration d'un flux préétabli d'air avec la phase d'expiration.

3. Appareil selon la revendication 1, **caractérisé en ce que** les moyens pour transporter le flux d'air dans les voies aériennes d'un patient (15) comprennent un connecteur et un masque ou un embout buccal, lesdits moyens pour transporter un flux préétabli d'air étant reliés fonctionnellement audit connecteur ou audit masque ou embout buccal par le biais des moyens pour transporter le flux d'air.
